# EUROPÄISCHE PATENTSCHRIFT

(11) **EP 1 383 899 B1**
(45) Veröffentlichungstag und Bekanntmachung des Hinweises auf die Patenterteilung: **23.09.2009**
(21) Anmeldenummer: 02737953.6
(22) Anmeldetag: 15.04.2002
(51) Int. Cl.: C12N 15/53, C12N 9/04, C12N 1/21, C12P 7/02

(54) **ENZYMATISCHES VERFAHREN ZUR ENANTIOSELEKTIVEN REDUKTION VON KETOVERBINDUNGEN**
ENZYMATIC METHOD FOR THE ENANTIOSELECTIVE REDUCTION OF KETO COMPOUNDS
PROCEDE ENZYMATIQUE DE REDUCTION ENANTIOSELECTIVE DE COMPOSES CETONIQUES

(30) Priorität: 20.04.2001 DE 10119274
(43) Veröffentlichungstag der Anmeldung: 28.01.2004
(73) Patentinhaber: IEP GmbH, 65203 Wiesbaden (DE)
(72) Erfinder: GUPTA, Antje, 65207 Wiesbaden (DE); BREESE, Klaus, 79115 Freiburg (DE); BANGE, Gert, 06108 Halle (DE); NEUBAUER, Peter, FIN-90800 Oulu (FI)
(74) Vertreter: Schwarz, Albin
(86) Internationale Anmeldenummer: PCT/EP2002/004143
(87) Internationale Veröffentlichungsnummer: WO 2002/086126

(56) Entgegenhaltungen:
- EP-A- 0 796 914
- WO-A-96/38577
- US-A- 5 342 767
- HUMMEL W: "NEW ALCOHOL DEHYDROGENASES FOR THE SYNTHESIS OF CHIRAL COMPOUNDS" ADVANCES IN BIOCHEMICAL ENGINEERING, BIOTECHNOLOGY, SPRINGER, BERLIN, DE, Bd. 58, 1997, Seiten 145-184, XP000677754 ISSN: 0724-6145
- BRANDSHAW C W ET AL: "ENZYMATIC SYNTHESIS OF (R) AND (S) 1-DEUTEROHEXANOL" APPLIED BIOCHEMISTRY AND BIOTECHNOLOGY, CLIFTON, NJ, US, Bd. 33, Nr. 1, 1. April 1992 (1992-04-01), Seiten 15-24, XP002053169 ISSN: 0273-2289
- BRUCE L J ET AL: "SOLVENT SELECTION STRATEGIES FOR EXTRACTIVE BIOCATALYSIS" BIOTECHNOLOGY PROGRESS, Bd. 7, Nr. 2, 1991, Seiten 116-124, XP002237300 ISSN: 8756-7938 in der Anmeldung erwähnt
- JONSSON ASA ET AL: "Thermodynamic and kinetic aspects on water vs. organic solvent as reaction media in the enzyme-catalysed reduction of ketones." BIOCHIMICA ET BIOPHYSICA ACTA, Bd. 1430, Nr. 2, 19. März 1999 (1999-03-19), Seiten 313-322, XP002237301 ISSN: 0006-3002
- HUMMEL WERNER: "Large-scale applications of NAD(P)-dependent oxidoreductases: Recent developments." TRENDS IN BIOTECHNOLOGY, Bd. 17, Nr. 12, Dezember 1999 (1999-12), Seiten 487-492, XP002237302 ISSN: 0167-7799

## Beschreibung

Die vorliegende Erfindung betrifft ein enzymatisches Verfahren zur enantioselektiven Reduktion von organischen Ketoverbindungen zu den entsprechenden chiralen Hydroxyverbindungen, eine Alkohol-Dehydrogenase aus Lactobacillus minor und ein enzymatisches Verfahren zur enantioselektiven Gewinnung von (S)-Hydroxyverbindung aus einem Razemat.

Optisch aktive Hydroxyverbindungen sind wertvolle Synthesebausteine zur Herstellung einer Vielzahl pharmakologisch wichtiger Verbindungen. Diese Verbindungen sind oft schwer herstellbar durch klassische chemische Verfahren und können die für pharmakologische Anwendungen geforderte Enantiomerenreinheit nur selten erreichen. Daher werden zur Herstellung chiraler Verbindungen in der Regel biotechnologische Verfahren angewendet, wobei die stereoselektive Reaktion entweder von ganzen Mikroorganismen oder mit isolierten Enzymen durchgeführt wird.

Dabei hat sich oft der Einsatz von isolierten Enzymen als vorteilhaft erwiesen, da mit solchen in der Regel höhere Ausbeuten sowie eine höhere Enantiomerenreinheit erzielbar sind.

Dehydrogenasen und insbesondere Alkohol-Dehydrogenasen sind wertvolle Katalysatoren zur Gewinnung von chiralen Produkten durch stereoselektive Reduktion von organischen Ketoverbindungen zu den entsprechenden chiralen Alkoholen. Bekannt sind im wesentlichen entsprechende Enzyme aus Hefe, Pferdeleber oder Thermoanaerobium brockii. Diese Enzyme benötigen als Coenzym NADH (Nicotinadenindinukleotid) oder NADPH (Nicotinadenindinukleotidphosphat). Weitere bekannte Alkohol-Dehydrogenasen sind beispielsweise eine (S)-spezifische Alkohol-Dehydrogenase aus Rhodococcus erythropolis oder eine (R)-spezifische Alkohol-Dehydrogenase aus der Gattung Lactobacillus. Beide Enzymtypen haben ein breites Substratspektrum an Ketoverbindungen und weisen eine hohe Enantioselektivität auf. Die Alkohol-Dehydrogenasen aus Lactobacillus kefir (DE 40 14 573) und Lactobacillus brevis (DE 196 10 984) eignen sich insbesondere zur Gewinnung von chiralen (R)-Alkoholen.

Nachteilig für die Anwendung von Alkohol-Dehydrogenasen sind allerdings die geringe Enzymstabilität und Enzymaktivität der Alkohol-Dehydrogenasen in organischen Lösungsmitteln und die oft nur geringe Wasserlöslichkeit der zu reduzierenden Ketoverbindungen. Ferner ist ein weiterer limitierender Faktor für die Anwendung der Alkohol-Dehydrogenasen in organischen Lösungsmitteln der notwendige Einsatz von NADP oder NAD als Cofaktorbedarf, da der Cofaktor (NADP, NAD) wasserlöslich ist und in ökonomischen Verfahren regeneriert wird.

Die Erfindung bezweckt durch Modifikation der Verfahrensbedingungen die genannten Nachteile zu verbessern. Diese Aufgabe wird erfindungsgemäß gelöst durch die Verwendung eines Zwei-Phasen-Systems, enthaltend ein organisches Lösungsmittel, Alkohol-Dehydrogenase, Wasser, Cofaktor und Ketoverbindung.

Das erfindungsgemäße Verfahren weist eine hohe Standzeit auf durch die enzymstabilisierende Wirkung des Lösemittels, eine enantiomeren Reinheit von mehr als 99,9 % der hergestellten chiralen Hydroxyverbindungen und eine hohe Ausbeute bezogen auf die eingesetzte Menge der Ketoverbindung.

Das erfindungsgemäße Verfahren betrifft daher ein Verfahren zur enantioselektiven Reduktion einer Ketoverbindung der Formel I

**R¹-C(O)-R²** **(I)**

wobei R¹ und R² unabhängig voneinander gleich oder verschieden sind und für
1. Wasserstoffatom,
2. -(C₁-C₂₀)-Alkyl, worin Alkyl geradkettig oder verzweigt ist,
3. -(C₂-C₂₀)-Alkenyl, worin Alkenyl geradkettig oder verzweigt ist und gegebenenfalls ein, zwei, drei oder vier Doppelbindungen enthält,
4. -(C₂-C₂₀)-Alkinyl, worin Alkinyl geradkettig oder verzweigt ist und gegebenenfalls ein, zwei, drei oder vier Dreifachbindungen enthält,
5 -(C₆-C₁₄)-Aryl,
6. -(C₁-C₈)-Alkyl-(C₆-C₁₄)-Aryl, oder
7. R¹ und R² bilden zusammen mit dem -C(O)-Rest ein -(C₆-C₁₄)-Aryl oder einen -(C₅-C₁₄)-Heterocyclus,
   wobei die oben unter 1. bis 7. genannten Reste unsubstituiert sind oder ein- bis dreifach substituiert sind unabhängig voneinander durch
   a) -OH,
   b) Halogen, wie Fluor, Chlor, Brom oder Jod,
   c) -NO₂,
   d) -C(O)-O-(C₁-C₂₀)-Alkyl, worin Alkyl gerade oder verzweigt ist und unsubstituiert oder ein- bis dreifach substituiert ist durch Halogen, Hydroxyl, Amino oder Nitro, oder
   e) -(C₅-C₁₄)-Heterocyclus, der unsubstituiert oder ein- bis dreifach substituiert ist durch Halogen, Hydroxyl, Amino oder Nitro,
   dadurch gekennzeichnet, dass man
   a) die Verbindung der Formel I, Alkohol-Dehydrogenase, Wasser, Cofaktor und ein organisches Lösungsmittel mit einem IogP von 0,5 bis 4,0 ,
   b) in einem Zwei-Phasen-System inkubiert und
   c) die gebildete chirale Hydroxyverbindung isoliert.

Kohlenstoffatomen im Ring. -(C₆-C₁₄)-Arylreste sind beispielsweise Phenyl, Naphthyl. Unter dem Begriff Aryl werden aromatische Kohlenstoffreste verstanden mit 6 bis 14 zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste. Unter dem Begriff "Halogen" wird ein Element aus der Reihe Fluor, Chlor, Brom oder Jod verstanden. Unter dem Begriff "-(C₁-C₂₀)-Alkyl wird ein Kohlenwasserstoffrest verstanden, dessen Kohlenstoffkette geradkettig oder verzweigt ist und 1 bis 20 Kohlenstoffatome enthält.

Der Begriff "-(C₅-C₁₄)-Heterocyclus" steht für einen monocyclischen oder bicyclischen 5-gliedrigen bis 14-gliedrigen heterocyclischen Ring, der teilweise gesättigt oder vollständig gesättigt ist. Beispiele für Heteroatome sind N, O und S. Beispiele für die Begriffe -(C₅-C₁₄)-Heterocyclus sind Reste, die sich von Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolone, Isoxazolone, Oxadiazolidindione, Triazole, welche durch F, -CN, -CF₃ oder -C(O)-O-(C₁-C₄)-Alkyl substituert sind, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen ableiten. Insbesondere bevorzugt sind die-Reste 2- oder 3-Pyrrolyl, Phenylpyrrolyl wie 4-oder 5-Phenyl-2-pyrrolyl, 2-Furyl, 2-Thienyl, 4-Imidazolyl, Methyl-imidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3-oder 5-Indolyl, substituiertes 2-Indolyl, zum Beispiel 1-Methyl-, 5-Methyl-, 5- Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzo-thienyl, 2-Benzoxazolyl oder Benzothiazolyl oder Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl oder Benzodioxolanyl.

Bevorzugte Verbindungen der Formel I sind 4-Chlor-3-oxo- butansäureethylester, Acetophenon, Acetessigsäuremethylester, Ethyl-2-oxo-4-phenylbutyrat, 2,5-Hexandion Ethylpyruvat oder 2-Octanon, bevorzugt 4-Chlor-3-oxo-butansäureethylester. Die Verbindungen der Formel I werden im erfindungsgemäßen Verfahren in einer Menge von 10% bis 25% bezogen auf das Gesamtvolumen eingesetzt, insbesondere von 15 % bis 22 %.

Dem Wasser wird bevorzugt ein Puffer zugesetzt, beispielsweise Kaliumphosphat-Tris/HCl- oder Triethanolamin-Puffer mit einem pH-Wert von 5 bis 10, vorzugsweise ein pH-Wert von 6 bis 9. Die Pufferkonzentration beträgt von 10 mM bis 150 mM, bevorzugt von 90 mM bis 110 mM, insbesondere 100 mM. Zusätzlich enthält der Puffer auch Magnesiumionen, beispielsweise MgCl₂ in einer Konzentration von 0,2 mM bis 10 mM, bevorzugt 0,5 bis 2 mM, insbesondere 1 mM.

Die Temperatur beträgt beispielsweise von etwa 10°C bis 70 °C, bevorzugt von 30 °C bis 60 °C.

Die erfindungsgemäß einsetzbaren organischen Lösungsmittel haben bevorzugt einen IogP von 0,6 bis 2,0, insbesondere von 0,6 bis 1,9 , insbesondere bevorzugt von 0,63 bis 1,75. Die bevorzugten organischen Lösungsmittel sind beispielsweise Diethylether, tertiär-Butylmethylether, Diisopropylether, Dibutylether oder Essigsäureethylester, insbesondere Essigsäureethylester. Essigsäureethylester kann beispielsweise in einer Menge von 1 % bis 90 % bezogen auf das Gesamtvolumen des Reaktionsansatzes eingesetzt werden, vorzugsweise von 15 % bis 60 %, insbesondere von 20 % bis 50 %.

Das Verhältnis von organischen Lösungsmittel zu Wasser beträgt von 9 zu 1 bis 1 zu 9, vorzugsweise von 1 zu 1 bis 1 zu 3.

Das Wasser bildet im erfindungsgemäßen Zwei-Phasen-System die eine flüssige Phase und das organische Lösungsmittel bildet die zweite flüssige Phase. Gegebenenfalls kann auch noch eine feste oder weitere flüssige Phase vorliegen, die beispielsweise durch nicht vollständig gelöste Alkohol-Dehydrogenase oder durch die Verbindung der Formel I entsteht. Bevorzugt sind jedoch zwei flüssige Phasen ohne feste Phase. Die zwei flüssigen Phasen werden bevorzugt mechanisch gemischt, so dass große Oberflächen zwischen den beiden flüssigen Phasen erzeugt werden.

Die Konzentration des Cofaktors NADPH oder NADH bezogen auf die wäßrige Phase beträgt von 0,05 mM bis 0,25 mM, insbesondere von 0,06 mM bis 0,2 mM.

Bevorzugt wird im erfindungsgemäßen Verfahren noch ein weiterer Stabilisator der Alkohol-Dehydrogenase eingesetzt. Geeignete Stabilisatoren sind beispielsweise Glycerin, Sorbitol oder Dimethylsulfoxid (DMSO).

Die Menge an Glycerin beträgt von 5 % bis 30 % bezogen auf das Volumen des gesamten Ansatzes. Bevorzugte Mengen an Glycerin sind von 10 % bis 20 %, insbesondere 20 %.

Zu Regenerierung des verbrauchten NADH oder NADPH kann im erfindungsgemäßen Verfahren zusätzlich Isopropanol zugefügt werden. Beispielsweise wird das Isopropanol und NADP mit der Alkohol-Dehydrogenase zu NADPH und Aceton umgesetzt. Die eingesetzte Isopropanolmenge beträgt von 5 % bis 30 % bezogen auf das Volumen des gesamten Ansatzes. Bevorzugte Mengen an Isopropanol sind von 10 % bis 20 %, insbesondere 10 %.

Geeignete Alkohol-Dehydrogenasen stammen beispielsweise aus Hefe, Pferdeleber oder Rhodococcus erythropolis, wobei diese Enzyme als Coenzym NADH benötigen, oder aus Thermoanaerobium brockii, Lactobacillus kefir oder Lactobacillus brevis, wobei diese Enzyme als Coenzym NADPH benötigen.

Wird eine Alkohol-Dehydrogenasen beispielsweise aus Hefe, Pferdeleber, Thermoanaerobium brockii oder Rhodococcus erythropolis im erfindungsgemäßen Verfahren eingesetzt, so wird aus der Verbindung der Formel I die entsprechende (S)-Hydroxyverbindung gewonnen. Wird eine Alkohol-Dehydrogenasen beispielsweise aus Lactobacillus kefir oder Lactobacillus brevis im erfindungsgemäßen Verfahren eingesetzt, so wird aus der Verbindung der Formel I die entsprechende (R)-Hydroxyverbindung gewonnen.

Die Alkohol-Dehydrogenase kann in dem erfindungsgemäßen Verfahren entweder vollständig gereinigt oder teilweise gereinigt eingesetzt werden oder in Zellen enthaltend verwendet werden. Die eingesetzten Zellen können dabei nativ, permeabilisiert oder lysiert vorliegen.

Die Volumenaktivität der eingesetzten Alkohol-Dehydrogenase beträgt von 100 Units/ml (U/ml) bis 2000 U/ml, bevorzugt etwa 800 U/ml, bei einem Proteingehalt von etwa 20 mg/ml bis 22 mg/ml. Die bevorzugt eingesetzte Alkohol-Dehydrogenase hat eine spezifische Aktivität von etwa 35 bis 40 U/mg Protein. Je kg umzusetzender Verbindung der Formel I werden 20 000 bis 200 000 U Alkohol-Dehydrogenase eingesetzt, bevorzugt etwa 100 000 U. Der Enzymeinheit 1 U entspricht dabei der Enzymmenge die benötigt wird um 1 µmol der Verbindung der Formel I je Minute (min) umzusetzen.

Das erfindungsgemäße Verfahren wird beispielsweise in einem geschlossenen Reaktionsgefäß aus Glas oder Metall durchgeführt. Dazu werden die Komponenten einzeln in das Reaktionsgefäß überführt und Rühren unter einer Atmosphäre von beispielsweise Stickstoff oder Luft gerührt. Je nach Substrat und eingesetzter Verbindung der Formel I beträgt die Reaktionszeit von 1 Tag bis 14 Tage, bevorzugt 4 bis 7 Tage.

Anschließend wird das Reaktionsgemisch aufgearbeitet. Dazu wird die wäßrige-Phase abgetrennt, die Essigsäureethylester-Phase wird gefiltert. Die wäßrige Phase kann gegebenenfalls noch einmal extrahiert werden und wie die Essigsäureethylester-Phase weiter aufgearbeitet werden. Danach wird die gefilterte Phase unter vermindertem Druck verdampft. Man erhält so beispielsweise das Produkt 4-Chlor-3-(S)-hydroxy-butansäureethylester mit einer Enantiomerenreinheit von mehr als 99,9% und im wesentlichen frei vom Edukt 4-Chlor-3-oxo- butansäureethylester. Die Gesamtausbeute der Prozesse beträgt nach Destillation des Produktes von 82 % bis 88% bezogen auf die eingesetzte Eduktmenge.

Überraschenderweise zeigen die organischen Lösungsmittel mit einem log-P-Wert von 0 bis 4 eine stabilisierende Wirkung auf die Alkohol-Dehydrogenase, während im Stand der Technik von der Verwendung der Zwei-Phasen-Systeme mit organischen Lösungsmittel abgeraten wird (M.R. Kula, U. Kragel; Kapitel 28, Dehydrogenases in Synthesis of Chiral Compounds; R. N. Patel, Stereoselective Biocatalyses, 2000; Peters J. 9. Dehydrogenases-Characteristics, Design of Reaction Conditions, and Application, In: H.J. Rehm, G. Reed Biotechnology, Vol 3, Bioprocessing, VCH Weinheim, 1993; J. Lynda et al. ; Solvent selection strategies for extractive Biocatalysis, Biotechnol. Prog. 1991, 7, Seiten 116- 124). Im erfindungsgemäßen Verfahren wird als organische Phase Essigsäureethylester verwendet , wobei die organische Phase zum einen als Reservoir für die Verbindung der Formel I dient, aber auch gleichzeitig das Reaktionsprodukt, die chirale Hydroxyverbindung aus der wäßrigen Phase extrahiert.

Im Gegensatz zum Stand der Technik führt der Einsatz von organischen Lösungsmitteln mit einem log-P-Wert von 0 bis 3 zu einer zusätzlichen im Zeitverlauf zunehmenden Stabilisierung der Alkohol-Dehydrogenase. Im Stand der Technik üben insbesondere organische Lösungsmittel mit einem log-P-Wert (Logarithmus des Octanol/ Wasser- Verteilungskoeffizienten) von 0 bis 2 eine besonders instabilisierende Wirkung auf Enzyme aus und kommen somit als organische Phase im Zwei-Phasen-System kaum in Betracht (K. Faber, Biotransformations in organic chemistry, 3rd edition 1997, Springer Verlag, Kapitel 3. Bis 3.17).

Die Erfindung betrifft ferner die Alkohol-Dehydrogenase aus Lactobacillus minor mit hohem Temperaturoptimum. Die Alkohol-Dehydrogenase aus Lactobacillus minor hat die DNA-Sequenz gemäß SEQ ID NO: 3 und die Aminosäuresequenz gemäß SEQ ID NO: 4 gemäß des beiliegenden Sequenzprotokolls. Diese Alkohol-Dehydrogenase aus Lactobacillus minor ist R-spezifisch, wobei beispielsweise aus einer Verbindung der Formel I die entsprechende (R)-Hydroxyverbindung gewonnen werden kann. Die enantioselektive Alkohol-Dehydrogenase aus Lactobacillus minor lässt sich überraschenderweise in Escherichia coli RB 791 überexprimieren, während Alkohol-Dehydrogenasen aus anderen Arten der Gattung Lactobacillus nur in wesentlich geringerer Weise exprimiert werden konnten. Dies ist um so überraschender, da die Alkohol-Dehydrogenase im Wildstamm von Lactobacillus minor selbst nur sehr gering exprimiert wird und somit mit gängigen Screeningverfahren (Ganzzellbiotransformation, Aktivitätstest) nicht nachweisbar war. Es war daher sehr überraschend, dass sich aus Lactobacillus minor eine R-enantioselektive Alkohol-Dehydrogenase klonieren ließ und in Escherichia coli so außerordentlich stark überexprimierbar war (50% des Zellproteins des Klons, 20.000 Units/g Feuchtgewicht).

Das gereinigte Enzym aus Lactobacillus minor ist stabil in einem pH-Bereich von etwa 5,5 bis 8,5. Das Enzym ist bis etwa 40 °C stabil und das pH-Optimum der enzymatischen Reaktion liegt im Bereich von pH 7 bis pH 7,5. Das Temperaturoptimum der enzymatischen Reaktion liegt bei etwa 55 °C. das Enzym weist ein breites Substratspektrum auf.

Das Enzym läßt sich mittels hydrophober Interaktionschromatographie bis zu einer spezifischen Aktivität von 35 bis 40 U/mg Protein reinigen.

Die Erfindung betrifft auch ein Verfahren zur Gewinnung der Alkohol-Dehydrogenase aus Lactobacillus minor. Dazu wird die DNA, die für die Alkohol-Dehydrogenase aus Lactobacillus minor kodiert, in einem geeigneten prokaryotischen oder eukaryotischen Mikroorganismus exprimiert. Bevorzugt wird die Alkohol-Dehydrogenase aus Lactobacillus minor in einen Escherichia coli Stamm transformiert und exprimiert, insbesondere in Escherichia coli RB 791.

Die Alkohol-Dehydrogenase aus Lactobacillus minor läßt sich beispielsweise so gewinnen, dass die rekombinanten Escherichia coli Zellen kultiviert werden, die Expression der Alkohol-Dehydrogenase induziert wird und anschließend nach etwa 10 bis 18 Stunden (h) die Zellen durch Ultraschallbehandlung oder durch French-Press (Gaullin, Siemens) aufgeschlossen werden. Der erhaltene Zellextrakt kann entweder direkt verwendet werden oder weiter gereinigt werden. Dazu wird der Zellextrakt beispielsweise zentrifugiert und der erhaltene Überstand wird einer hydrophoben Interaktionschromatographie unterworfen. Diese Chromatographie erfolgt bevorzugt bei pH 7,0 in einem wässrigen Puffer der auch Magnesiumionen enthält.

Die erfindungsgemäße Alkohol-Dehydrogenase kann für die Herstellung der Verbindung der Formel II entweder vollständig oder teilweise gereinigt vorliegen oder kann auch enthaltend in Zellen im Verfahren eingesetzt werden. Die Zellen können dabei nativ, permeabilisiert oder lysiert vorliegen.

Gegenstand der Erfindung ist auch ein rekombinanter Klon von Escherichia coli RB 791, der die Alkohol-Dehydrogenase aus Lactobacillus minor exprimiert und am 26. März 2001 unter den Bedingungen des Budapester Vertrages bei der Deutschen Sammlung für Mikroorganismen und Zellkulturen, Mascheroder Weg 1 b, 38124 Braunschweig unter der Nummer DSM 14196 hinterlegt wurde.

Die Erfindung wird durch die folgenden Beispiele erläutert:

### Beispiel 1

### Screening nach R-Alkohol-Dehydrogenasen in Stämmen der Gattung Lactobacillus mittels Ganzzellbiotransformation

Zum Screening wurden verschiedene Lactobacillenstämme in folgendem Medium kultiviert (Angaben jeweils g/I): Glucose (20), Hefeextrakt (5), Fleischextrakt (10), Di-Ammoniumhydrogencitrat (2), Natriumacetat (5), Magnesiumsulfat (0,2), Mangansulfat (0,05), Di-Kaliumhydrogenphosphat (2).

Das Medium wurde bei 121°C sterilisiert und die Stämme der Gattung Lactobacillus (im folgenden mit L. abgekürzt) wurden ohne weitere pH-Regulierung oder Sauerstoffzufuhr kultiviert. Anschließend wurden die Zellen abzentrifugiert und für die Ganzzellbiotransformation wurden jeweils 4g Zellen in einem Endvolumen von 10 ml Kaliumphosphatpuffer (KPi-Puffer) (50 mM, pH = 7.0) resuspendiert. Nach Zugabe von je 0,1 g Glucose wurden die Zellen für 15 min bei 30 °C geschüttelt. Zur Zellsuspension wurde 4-Chlor-3-oxo- butansäureethylester 4 in einer Endkonzentration von 40 mM zugegeben und nach jeweils 10 min und 120 min erfolgte die gaschromatographische Analyse des Medium. Dazu wurden die Zellen abzentrifugiert, der Überstand filtriert und in Chloroform bis zu einer Endkonzentration von 10-15 µg/ml 4-Chlor-3-oxo-butansäureethylester verdünnt.

Der als Substrat eingesetzte 4-Chlor-3-oxo-butansäureethylester wurde von den verschiedenen Lactobacillenstämmen mit folgender Enantiomerenreinheit zum Ethyl (S)-4 chloro-3-hydroxybutyrat umgesetzt.

Der Enantiomerenüberschuß berechnet sich wie folgt: ee(%) = ((R-Alkohol -S-Alkohol)/(R-Alkohol+ S-Alkohol)) x 100.

**Tabelle 1**

| Stamm Lactobacillus | ee 4-Chlor-3-(S)-hydroxy-butansäureethylester in % |
|---|---|
| L. reuteri | 34,6 |
| L. kandleri | 90,0 |
| L. collinoides | 71,3 |
| L. bifermentans | 53,6 |
| L. oris | 63,4 |
| L. brevis | 74,0 |
| L. halotolerans | 67,2 |
| L. minor | 18,6 |
| L. parabuchneri | 78,5 |
| L. kefir | 87,8 |
| L. fructosus | 28,9 |

### Beispiel 2

### Gewinnung rekombinanter R-spezifischer Alkohol-Dehydrogenasen

### A.) Präparation genomischer DNA aus Stämmen der Gattung Lactobacillus

Das Zellpellet aus etwa 2 ml Kulturflüssigkeit der Gattung Lactobacillus wurde in 300 µl TE-Puffer (enthaltend 10 mM Tris/HCl, pH = 8, 1 mM EDTA) resuspendiert, mit 20 mg/ml Lysozym versetzt und 10 min bei 37°C inkubiert. Anschließend wurden 100 µl Natriumdodecylsulfat (SDS) (10%), 100µl Na-Perchlorat (5M) und 500 µl Chloroform/Isoamylalkohol (24:1) zugegeben. Nach kräftigem Schütteln wurde das Protein abzentrifugiert und die wäßrige Phase in ein neues Eppendorfgefäß überführt. Danach wurden 800 µl Ethanol (EtOH) (96 %) zugegeben. Das Eppendorfgefäß wurde mehrfach invertiert und anschließend die ausgefallene chromosomale DNA in ein neues Eppendorfgefäß überführt und mit 200 µl ETOH gewaschen. Die DNA wurde wiederum in ein neues Eppendorfgefäß überführt, unter verminderten Druck getrocknet und in 100µl TE-Puffer gelöst.

### B.) Oligonukleotide als 5'- und 3'-Primer für die PCR (Polymerase chain reaction)

Die für die PCR verwendeten Primer wurden von der bekannten N-terminalen und C-terminalen Sequenz der Alkohol-Dehydrogenase aus L. kefir abgeleitet. Dabei wurden bekannte Präferenzen für bestimmte Codons in Lactobacillen berücksichtigt. So wurde vor jeden 5'-Primer das Codon ATG (Met) als Startcodon vorgesetzt, weiterhin wurde am 5'-Primer dem Startcodon die Schnittstelle für das Restriktionsenzym Bam HI (GGATCC) vorangestellt um die spätere Klonierung in den Expressionsektor zu ermöglichen. Hinter den 3'-Primer wurde das Stop codon (TAG) und die Schnittstelle
für Hind III (AAGCTT) gesetzt. Die Primerkonstrukte sind im folgenden aufgelistet
N = A,T, C oder G; Y = T oder C; R = A oder G
5'Primer
   5'GCGGATCCATGACNGAYCGNTTRAARGGNAARGTNGC3' (SEQ ID NO:1)
3'Primer
   5'GGGAAGCTTCTAYTGNGCNGTRTANCCNCCRTCNAC3' (SEQ ID NO:2)

Die Primer wurden nach bekannten Verfahren hergestellt.

### C.) PCR (Polymerase chain reaction) mit der genomischen DNA aus Stämmen der Gattung Lactobacillus

PCR-Ansatz (100µl):

| | Einsatz pro Reaktion | Konzentration |
|---|---|---|
| dNTP's | 8µl | je NTP 2.5 nmol/µl |
| Oligos | je Oligo 10µl: 20µl | 2pmol/µl |
| chromosomale DNA | 3µl | ca. 1µg/µl |
| 10 x Puffer (Promega) | 10µl | |
| Taq- Polymerase (Promega) | 1µl | 2U/µl |
| H₂O | 58 µl | |

dNTP's sind ein Gemisch von Desoxynucleotidtriphosphaten wie dATP, dGTP, dCTP,dTTP

### Zyklus:

95 °C 2 min, danach
80 °C halten
   heißer Start, danach
95 °C 30 sec, danach
40 °C 1 min 30x
   danach jeweils 30 mal 95 °C 30 sec, und 40 °C 1 min, anschließend
72 °C 2,5 min
   danach
72 °C 2,5 min
   danach
10 °C halten

Für die Analytik wurden 10 µl des Ansatzes auf ein 1 %iges Agarosegel aufgetragen und bei konstant 100 V elektrophoretisch aufgetrennt. Die PCR zeigte die deutliche Amplifikation eines DNA-Stückes von etwa 750 bp.

### D.) Isolierung der PCR-Fragmente aus dem Gel

Zur Gewinnung des PCR-Fragments wurde der gesamte PCR-Ansatz auf ein 1 %iges Agarosegel aufgetragen und bei konstant 1 00V elektrophoretisch aufgetrennt. Dazu wurde das Gel in zwei Spuren geteilt, wovon eine den kompletten PCR-Ansatz und die andere nur eine Probe von 5 µl enthielt, so dass zum Ausschneiden des PCR-Fragments aus dem Gel zur Orientierung nur die Spur mit der Probe mit Ethidiumbromid angefärbt wurde um eine Schädigung des zu isolierenden PCR-Fragment durch Ethidiumbromid und durch UV-Licht auszuschließen.

Die Isolierung aus dem Gel erfolgte mit dem Q1Aquick Gel Extraction Kit von der Firma Qiagen aus Hilden.

Die Konzentrationsbestimmung ergab eine Gesamtkonzentration von 20 ng/µl DNA.

### E.) Ligation

Zur Vorbereitung der Ligation wurden das gereinigte PCR-Fragment und der verwendete Klonierungsvektor pQE30 oder pQE 70 beide von der Firma Quiagen mit Bam HI und Hind III geschnitten (4µl DNA= 200ng DNA, 1µl 10xPuffer; 1µl Enzym, BSA und H₂O (Biolabs, New England)).

Das geschnittene Plasmid wurde dann erneut mittels OIAquick Gel Extraction Kit gereinigt, in Wasser aufgenommen mittels alkalischer Phosphatase dephosphoryliert (USB, Amersham Life Science).

Zur Reinigung wurden die .entsprechenden Reaktionsansätze erneut auf ein 1%iges Agarosegel aufgetragen und so das verdaute Amplifikat sowie das Plasmid wie unter D.) beschrieben aus dem Gel isoliert. Die Konzentration von Plasmid und Amplifikat nach der Reinigung betrug etwa 20 ng/µl.

Zur Ligation wurden 3 µl pQE30 oder pQE 70 (60ng), 2,5µl Amplifikat (50ng), 2µl Ligasepuffer (Boehringer; Mannheim), 1,5 µl H₂O und 1µl T4-Ligase (Boehringer; Mannheim) eingesetzt. Der Ansatz wurde über Nacht bei 16 °C inkubiert.

Anschließend wurden 40 µl elektrokompetente Zellen von Escherichia coli RB791 mit 1,5 µl Ligationsansatz durch Elektroporation transformiert. Die Zellen wurden in 500 µl SOC-Medium gegeben, 45 min bei 37 °C inkubiert und anschließend je 250 µl auf LBₐₘₚ- Agarplatten ausplattiert. Das SOC-Medium enthält pro Liter Wasser 20 g Trypton, 5 g Hefe-Extrakt, 0,5 g NaCl, 10 ml von 1 M MgSO₄ und 10 ml von 1 M MgCl₂. LBₐₘₚ-Agarplatten enthalten pro Liter Wasser 10 g Trypton, 5 g Hefe-Extrakt, 10 g NaCl, 20 g Agar, pH 7,0 und 50 mg Ampicillin.

Gewachsene Kolonien wurden abgeimpft und in 4ml Flüssigkultur (LBₐₘₚ-Medium) über Nacht bei 37 °C kultiviert. Von dieser Zellsuspension wurden je 2 ml zur Plasmidpräparation (entsprechend dem Quiagen miniprep Protokoll (Quiagen, Hilden)) eingesetzt.

Von der Plasmidpräparation wurden ein Restriktionsverdau mit Bam HI und HindIII angesetzt. Der komplette Verdau wurde auf ein 1 % iges Agarosegel aufgetragen und bei 100 V elektrophoretisch aufgetrennt (Nachweis des 750 kp Inserts) und die Plasmide daraufhin gegebenenfalls zur Sequenzierung eingesetzt. Klone mit 750 kp Insert wurden dann auf LBₐₘₚ-Agarplatten ausplattiert.

### F.) Sequenzierung der Plasmide

Die Sequenzierung wurde mittels dem SequiThermEXCEL II Long-Read DNA Sequencing Kit (Biozym, Oldendorf) am Li-Cor-Sequenzer (MWG Biotech, Ebersberg) entsprechend den Angaben des Herstellers durchgeführt. Als Primer wurden die Standard Sequenzierungsprimer für pQE-Vektoren benutzt.

### G.) Screening der Klone hinsichtlich löslicher Expression der R-ADH

Klone mit Inserts von 750 kp wurden hinsichtlich enzymatischer Aktivität und Stereoselektivität untersucht. Dazu wurden die Kolonien von den LBₐₘₚ-Agarplatten abgeimpft und in 20 ml Flüssigkulturen (LBₐₘₚ-Medium) bei 25 °C kultiviert. Bei einer Zelldichte (OD₅₀₀) von 0,5 erfolgte dann die Induktion mit 1 mM Isopropyl-ß-D-thiogalactopyranosid (IPTG). Nach 18 h wurden die Zellen abzentrifugiert und je 40 mg Zellen in 350 µl Kpi-Puffer (50mM, pH = 7, 1 mM MgCl₂) aufgenommen. Die Enzymfreisetzung aus den Zellen wurde durch Naßvermahlung mit Hilfe von Glasperlen (0,5 g, 0,3 mm) erreicht. Dazu erfolgte ein 20 minütlicher Aufschluß mittels Retsch-Mühle bei 4°C.

Der Enzymtest enthielt 870 µl Triethanolaminpuffer (100mM, pH=7,0, 1mM MgCl₂). 100 µl einer 100mmolaren Lösung 4-CI-Acetessigsäureethylester, 10 µl NADPH (Endkonzentration 0,19mM) und 20 µl Enzymlösung.

Die Definition der Enzymeinheit: 1 U entspricht der Enzymmenge die benötigt wird um 1 µmol Substrat (4-Chlor-3-oxo- butansäureethylester) pro 1 min umzusetzen.

Zum Nachweis der Stereoselektivität wurden 480 µl Triethanolaminpuffer (100mM; pH=7,0, 1 mM MgCl₂) mit 1,0 mM 4-Chlor-3-oxo-butansäureethylester, 1,9 mM NADPH (jeweils Endkonzentration) und 20 µl Enzymlösung inkubiert. Nach 15 min Inkubation wurde der Reaktionsansatz filtriert und 1:10 in Chloroform verdünnt und eine Probe wurde mittels GC-MS analysiert.

Bedingungen der Gaschromatographie (GC):
chirale Säule: Lipodex E, ID = 0,25mm, I = 25m (Macherey-Nagel)
   1. 2 min 60°C
   2. in 28 min von 60 °C auf 130 °C mit einer Rate von 2,5 °C pro Minute
   3. 15 min bei 130 °C

Aus den folgenden Lactobacillenstämmen konnte eine (R)-spezifische Alkohol-Dehydogenase kloniert und aktiv überexprimiert werden:

| Stamm | Plasmid | Klon Nummer | Aktivität in U/g Zellen* | ee in % |
|---|---|---|---|---|
| L. parabuchneri | pQE 30 | 12 | 450 | >99,9 |
| L. parabuchneri | pQE 30 | 14 | 170 | >99,9 |
| L. kandleri | pQE 30 | 11 | 280 | >99,9 |
| L. kandleri | pQE 70 | 17 | 710 | >99,9 |
| L. minor | pQE 30 | 2 | 2.830 | >99,9 |
| L. minor | pQE 70 | 3 | 680 | >99,9 |
| L. minor | pQE 70 | 4 | 700 | >99,9 |

| | | | | |
|---|---|---|---|---|
| * Aktivität berechnet aus G.) (Naßvermahlung); Die Aktivitäten liegen nach Fermentation und Aufschluß mit French-Press erheblich höher. | | | | |

### H.) Enzymgewinnung und Reinigung

Der Stamm mit der höchsten enzymatischen Aktivität wurde zur Enzymgewinnung im Fermenter ( Fed batch, 10 l) kultiviert. Die Induktion erfolgte bei einer OD₅₀₀ von 40 mit 1 mM IPTG. Nach 18 h erfolgte die Zellernte wobei 300 g Zellen in 3 I Kpi-Puffer (50mM, pH = 7, 1 mM MgCl₂) aufgenommen wurden, anschließend erfolgte der Zellaufschluß mittels French-Press (Gaullin, Siemens). Der nach Zentrifugation erhaltene Überstand wird im folgenden als Rohextrakt bezeichnet und wies eine Volumenaktivität von etwa 2000 U/ml (20 000 U/g Feuchtmasse) auf.

Für die Enzymcharakterisierung wurde ein Teil des erhaltenen Enzyms mittels hydrophober Interaktionschromatographie auf Q-Sepharose ff (fast flow) gereinigt. Die verwendete Säule wurde dazu mit 50mM Kpi-Puffer pH = 7.0, 1mM MgCl₂ äquilibriert. Nach Auftragen des Rohextrakts auf die Säule und kurzem Spülen mit dem Äquilibrierungspuffer wurde das Enzym mit einem steigenden linearem Salzgradienten (0-1 M NaCl, 1 ml/min) bei einer Salzkonzentration von etwa 0,3 M NaCl eluiert. Nach Vereinigen der enzymhaltigen Fraktionen erhielt man etwa 25 ml des gereinigten Enzyms mit einer Volumenaktivität von etwa 800 U/ml und einem Proteingehalt von 20 bis 22 mg/ml. Das so gereinigte Enzym hat also eine spezifische Aktivität von etwa 35 bis 40 U/mg Protein.

Alle enzymatischen Aktivitäten wurden bei 25 °C bestimmt. Die Enzymaktivität wurde wie folgt berechnet:
- Berechnung:: 1 Unit = 1µmol Substratumsatz/min
Lambert-Beersches Gesetz

Abnahme des NADPH wurde bei 340 nm verfolgt (siehe enzymatischer Testansatz) = ΔE/min
- N =: Verdünnungsfaktor Enzym
- V =: Volumen Enzym im ml (0,01)
- V_{küvette} =: Küvettenvolumen = 1 ml
- d =: Schichtdicke der Küvette = 1 cm
- e_{NADPH}=: Extinktionskoeffizient NADPH = 6,22[mM⁻¹ * cm⁻¹]

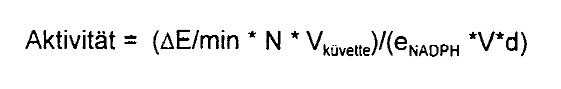

Proteinbestimmung wurde nach Bradford angewandt (Bio-Rad- Laboratories GmbH, Protein Assay)

### Beispiel 3

### Enzymkatalysierte Herstellung von Ethyl (S)-4 chloro-3-hydroxybutyrat A. ) im 5 Liter Maßstab

Für die enzymkatalysierte Synthese von Ethyl (S)-4 chloro-3-hydroxybutyrat aus 4-Chlor-3-oxo- butansäureethylester wurde der in Beispiel 2 gewonnene Rohextrakt der Alkohol-Dehydrogenase und das Coenzym NADP eingesetzt. Das oxidierte Coenzym wurde durch gleichzeitige Anwesenheit von Isopropanol kontinuierlich regeneriert, so dass die Reaktion nur katalytische Mengen an Coenzym erfordert.

Der Ansatz enthielt :
2 I Triethanolamin- Puffer 100mM pH =7.0, 1 mM MgCl₂, 10% Glycerin, 400 mg NADP,
600 ml Isopropanol,
800 ml Essigsäureethylester,
600 ml 4-Chlor-3-oxo- butansäureethylester und
   etwa 100 000 Units Alkohol-Dehydrogenase.

Nach 3 Tagen Rühren bei Raumtemperatur konnte gaschromatographisch der vollständige Umsatz des 4-Chlor-3-oxo-butansäureethylester zum Ethyl (S)-4 chloro-3-hydroxybutyrat mit über 99,9 % Enantiomerenreinheit nachgewiesen werden.

Nach Abtrennung der wässrigen Phase, Abdampfen des Lösungsmittels und gegebenenfalls Destillation erhält man das saubere Ethyl (S)-4 chloro-3-hydroxybutyrat mit über 99,9%iger Enantiomerenreinheit.

### B. ) im 50I Maßstab

Der Reaktionsansatz zum Umsatz von 10 I 4-Chlor-3-oxo- butansäureethylester ist wie folgt zusammengesetzt;:
18 I Triethanolamin- Puffer 100mM pH =7.0, 1 mM MgCl₂, 10% Glycerin,
4 g NADP,
10 l Isopropanol,
10 l Essigsäureethylester,
10l 4-Chlor-3-oxo- butansäureethylester und
   etwa 2 Millionen Units Alkohol-Dehydrogenase (1,25I Rohextrakt).

Nach 7 Tagen Rühren bei Raumtemperatur konnte gaschromatographisch der vollständige Umsatz des 4-Chlor-3-oxo-butansäureethylesters zum Ethyl (S)-4 chloro-3-hydroxybutyrat mit über 99,9 % Enantiomerenreinheit nachgewiesen werden.

### Beispiel 4

### Biochemische Charakterisierung der klonierten Alkohol-Dehydrogenase aus Lactobacillus minor

### A.) pH-Stabilität

Die Abhängigkeit der Aktivität des Enzyms bei Lagerung in Puffern mit verschiedenen pH-Werten wurde im Bereich von pH 4 bis 11 untersucht. Dazu wurden verschiedene Puffer (50mM) im Bereich von pH 4 bis 11 angesetzt und das in Beispiel 2 gereinigte Enzym darin 1:100 verdünnt und 30 min inkubiert. Alle Puffer enthielten 1mM MgCl₂. Anschließend wurden davon 10µl im normalen Enzymtest eingesetzt (Triethanolaminpuffer 100mM pH = 7.0, 1 mM MgCl₂. 10 mM 4-Chlor-3-oxo- butansäureethylester und 0,19mM NADPH). Die Reaktion wurde für 1min bei 30 °C und 340 nm verfolgt.

Ausgangswert ist dabei der Messwert, den man unmittelbar nach Verdünnung des Enzyms in Triethanolaminpuffer 50 mM pH = 7.0 erhält. Dieser Wert entsprach unter vorgegeben Bedingungen einer Extinktionsänderung von 0,20 /min und wurde als 100%-Wert gesetzt und alle folgenden Messwerte wurden zu diesem Wert ins Verhältnis gesetzt.

**Tabelle 2**

| PH-Wert | Puffersystem | Aktivität in % (n=2) | Puffersystem | Aktivität in % (n=2) |
|---|---|---|---|---|
| 4 | Na-acetat/ Essigsäure | 87,5 ± 6,5 | | |
| 4,5 | Na-acetat/ Essigsäure | 94,5 ± 3,0 | | |
| 5 | Na-acetat/ Essigsäure | 94.5 ±1,5 | MES/NaOH | 55 ± 5 |
| 5,5 | KH₂PO₄K₂PO₄ | 96 ± 3 | MES/NaOH | 77,1 ±2,1 |
| 6 | KH₂PO₄K₂PO₄ | 100 ± 0 | Triethanolamin/NaOH | 100±0 |
| 6,5 | KH₂PO₄K₂PO₄ | 97,5 ± 2,5 | Triethanolamin/NaOH | 100 ± 0 |
| 7 | KH₂PO₄/K₂PO₄ | 100 ± 0 | Triethanolamin/NaOH | 97,9 ±2,1 |
| 7,5 | KH₂PO₄K₂PO₄ | 97,5 ± 7,5 | Tris/HCl | 94,6 ±1,3 |
| 8 | KH₂PO₄K₂PO₄ | 93,0 ± 3,0 | Tris/HCl | 89,2 ± 0 |
| 8,5 | KH₂PO₄/K₂PO₄ | 102,5 ± 2,5 | Tris/HCl | 60 ± 4,2 |
| 9 | Glycin/NaOH | 76,5 ±1,5 | Tris/HCl | 63,1 ± 4,8 |
| 9,5 | Glycin/NaOH | 52,5 ± 7,5 | | |
| 10 | Glycin/NaOH | 52,5 ± 7,5 | | |
| 11 | Glycin/NaOH | 0,0 ± 0 | | |

Tabelle 2 zeigt, dass das Enzym eine gute pH-Stabilität insbesondere im sauren Bereich aufweist, dabei scheint die Enzymstabilität nicht nur vom pH-Wert, sondern auch vom verwendeten Puffersystem abhängig zu sein. Beispielsweise stellt man bei der Verwendung von TRIS und MES-Puffer bei gleichem pH-Wert eine stärkere Inaktivierung des Enzyms fest als im KPi-Puffer.
Im KPi-Puffer zeigte sich im pH-Bereich von 5,5 bis 8,5 keine signifikante Inaktivierung.

### B.) Temperaturstabilität

In analoger Weise wie unter A.) beschrieben wurde die Temperaturstabilität für den Bereich von 25 °C bis 50 °C bestimmt. Dazu wurde jeweils eine 1:100 Verdünnung des gereinigten Enzyms für 30 min bei der jeweiligen Temperatur inkubiert und anschließend bei 30.°C mit dem obigen Testansatz gemessen. Auch hier wurde als Ausgangswert der Meßwert herangezogen, den man unmittelbar nach Verdünnung des Enzyms in Triethanolaminpuffer 50 mM pH = 7.0 erhält. Dieser Wert wurde auch hier als 100%-Wert gesetzt. Die Alkohol-Dehydrogenase aus L. minor ist bis zu einer Temperatur von 40 °C stabil. Danach sinkt die Aktivität rapide ab.

**Tabelle 3**

| Temperatur | Aktivität in % (n=4) | Temperatur | Aktivität in % (n=4) |
|---|---|---|---|
| 25 | 101 ± 3,2 | 40 | 33,4 ± 3,8 |
| 30 | 81,2 ± 5,8 | 42 | 0 ± 0 |
| 35 | 67,0 ± 1,6 | 45 | 0 ± 0 |
| 37 | 20,2 ± 2,4 | 50 | 0 ± 0 |

### C.) pH-Optimum

Zur Bestimmung des pH-Optimums wurde die enzymatische Reaktion in dem jeweiligen in Tabelle 3 aufgeführten Puffer bestimmt. Die Konzentration des 4-Chlor-3-oxo- butansäureethylester betrug wie im Standardtest 10 mM und von NADPH 0,19 mM. Die Reaktion wurde bei 30 °C bestimmt. Dabei konnte für das erfindungsgemäße Enzym ein pH-Optimum zwischen 7 und 7,5 ermittelt werden.

**Tabelle 4**

| pH-Wert | Puffersystem | Aktivität in U/ml unverdünntes Enzym |
|---|---|---|
| 4 | Na-acetat/Essigsäure | 85 |
| 4,5 | Na-acetat/Essigsäure | 132 |
| 5 | MES/NaOH | 218 |
| 5,5 | MES/NaOH | 240 |
| 6 | Triethanolamin/NaOH | 381 |
| 6,5 | Triethanolamin/NaOH | 349 |
| 7 | Triethanolamin/NaOH | 510 |
| 7,5 | Tris/HCl | 707 |
| 8 | Tris/HCl | 585 |
| 8,5 | Tris/HCl | 486 |
| 9 | Tris/HCl | 488 |
| 10 | Glycin/NaOH | 131 |
| 11 | Glycin/NaOH | 0 |

### D.) Temperatur-Optimum

Zur Bestimmung der optimalen Testtemperatur wurde die Enzymaktivität von 25 °C bis 60 °C gemessen. Der Testansatz entsprach der Standardkonzentration von 4-Chlor-3-oxo- butansäureethylester und NADPH. Wie aus Tabelle 5 ersichtlich hat das Enzym seine optimale Testtemperatur bei 55 °C, anschließend sinkt die Aktivität rapide ab.

**Tabelle 5**

| Temperatur | Aktivität in U/ml unverdünntes Enzym | Temperatur | Aktivität in U/ml unverdünntes Enzym |
|---|---|---|---|
| 25 | 540 | 45 | 2469 |
| 30 | 1235 | 50 | 2469 |
| 35 | 1968 | 55 | 2855 |
| 40 | 1621 | 60 | 0 |

### E.) Substratspektrum

Ferner wurden anstelle von 4-Chlor-3-oxo-butansäureethylester noch weitere Substrate in dem enzymatischen Testansatz eingesetzt. Dafür wurde folgender Testansatz verwendet:
970 µl Triethanolaminpuffer (100 mM, pH = 7.0, 1 mM MgCl₂ mit 10mM Ketoverbindung)
20 µl NADPH (0,19mM im Testansatz)
10 µl Enzym (1:100)

Dabei wurde die mit 4-Chlor-3-oxo- butansäureethylester ermittelte Aktivität 100% gesetzt und die Enzymaktivitäten der anderen Substrate zu diesem Wert ins Verhältnis gesetzt.

**Tabelle 6**

| Substrat | Aktivität in % (n=2) |
|---|---|
| 4-Chlor-3-oxo- butansäureethylester | 100 |
| Ethylpyruvat | 192,3 ± 11,5 |
| 2-Octanon | 90,8 ± 1,2 |
| Acetoacetatmethylester | 120 ± 7,7 |
| 2-Oxo-4-phenylbutyratethylester | 62,7 ± 4,8 |

### F.) Enzymstabilität in organischen Lösungsmitteln

Zur Untersuchung der Enzymstabilität bei Kontakt mit organischen Lösungsmitteln wurde die Alkohol-Dehydrogenase aus L. minor in den angegebenen Löungsmitteigemischen 1: 100 verdünnt und bei Raumtemperatur inkubiert (bei nicht-wassermischbaren organischen Lösungsmitteln bezieht sich die Verdünnung auf die wäßrige Phase). Dabei wurde eine ständige Durchmischung beider Phasen gewährleistet (Shaker, 200rpm). Anschließend wurden 10 µl der Enzymlösung im Standardtestansatz eingesetzt. Auch hier wurde der Ausgangswert nach Verdünnung im Puffer (Triethanolaminpuffer 100mM, pH = 7.0, 1 mM MgCl₂) gleich 100 % gesetzt und alle weiteren Werte zu diesem ins Verhältnis gesetzt.

### Tabelle 7:

### A.) wassermischbare Lösungsmittel:

| **Lösungsmittel** | **IogP** | **t= 2h** | **T= 8h** | **t= 24h** | **t= 48h** |
|---|---|---|---|---|---|
| **Puffer** | | ***86*** | ***70*** | ***3*** | ***0*** |
| **10 % Isopropanol** | 0,28 | 32 | 34 | 16 | 0 |
| **20 % Isopropanol** | 0,28 | 16 | 17 | 7 | 0 |
| **10 % DMSO** | -1,3 | 73 | 54 | 60 | 40 |
| **20% DMSO** | -1,3 | 73 | 54 | 57 | 40 |
| **1M Sorbitol** | | 93 | 74 | 60 | 6 |
| **10 % Glyzerin** | -3,0 | 120 | 64 | 62 | 28 |
| **20 % Glyzerin** | -3,0 | 120 | 100 | 100 | 104 |

Wie aus Tabelle 7A ersichtlich wirken Glyzerin, DMSO und Sorbitol aktivierend bzw. stabilisierend auf die eingesetzte Alkoholdehydrogenase. Das im Prozess einzusetzende Isopropanol hingegen wirkt inaktivierend.

### B.) nicht wassermischbare Lösungsmittel

| **Lösungsmittel** | **LogP** | **t= 2h** | **t= 8h** | **t= 24h** | **t= 48h** |
|---|---|---|---|---|---|
| **Puffer** | | ***86*** | ***70*** | ***3*** | ***0*** |
| **20 % Essigsäureethylester** | 0,68 | 87 | 50 | 10 | 8 |
| **20 % Diethylether** | 0,85 | 53 | 42 | 37 | 23 |
| **20 %Tert-Buthylmethylether** | 1,21 | 67 | 51 | 38 | 24 |
| **20 Diisopropylether** | 1,55 | 100 | 57 | 41 | 29 |
| **20 % Dibutylether** | 2,9 | 92 | 71 | 23 | 6 |
| **20 % Pentan** | 3,0 | 74 | 55 | 7 | 6 |
| **20 % Hexan** | 3,5 | 80 | 39 | 2 | 5 |
| **20 % Heptan** | 4 | 51 | 49 | 7 | 6 |
| **20 % Octan** | 4,5 | 87 | 47 | 2 | 1 |

Wie aus Tabelle 7B ersichtlich zeigt die untersuchte Alkoholdehydrogenase in einer breiten Zahl organischer Lösungsmittel eine beachtliche Stabilität. Auffallend ist dabei, dass Lösungsmittel mit IogP- Werten zwischen 0 und 3 die untersuchte Alkoholdehydrogenase nicht stärker inhibieren als solche mit log P-Werten zwischen 3 und 4,5, insbesondere im Hinblick auf längere Inkubationszeiten (24 h und 48 h) haben Lösungsmittel mit log P-Werten zwischen 0 und 3 stabilisierende Wirkung auf die untersuchte ADH, verglichen mit den entsprechenden Werten im Puffer. Die untersuchten aliphatischen Lösungsmittel Pentan, Hexan, Heptan und Octan zeigen diese stabilisierende Wirkung bei Langzeitinkubation nicht.

Der log P-Wert einer KomponenteX ist der Logarithmus des Verteilungskoeffizient von X im Octanol/Wasser Zweiphasensystem (50/50)
P = Konzentration von X in Octanolphase/ Konzentration von X in wäßriger Phase

### G. Enzymstabilität unter Prozessbedingungen

Zur Untersuchung der Enzymstabilität unter Prozessbedingungen wurde die Alkohol-Dehydrogenase aus L. minor mit den im Zwei-Phasen-System verwendeten Löungsmittelgemischen 1 : 100 verdünnt und bei Raumtemperatur inkubiert.
Anschließend wurden 10 µl der Enzymlösung im Standardtestansatz eingesetzt.

In Tabelle 8 sind die Enzymaktivitäten in % vom Ausgangswert dargestellt.

**Tabelle 8**

| | 6 h | 20 h | 46 h | 60 h | 84 h |
|---|---|---|---|---|---|
| Triethanolamin-Puffer, 100 mM, 1 mM MgCl₂ | 100 | 75 | 0 | 0 | 0 |
| Mischung B | 100 | 85 | 80 | 60 | 55 |
| Mischung C | 110 | 95 | 95 | 85 | 80 |
| Mischung D | 100 | 65 | 55 | 50 | 50 |

Mischung B: Puffer, 10% Glycerin, 10% Isopropanol
Mischung C: Puffer, 20% Glycerin, 10% Isopropanol
Mischung D: Puffer, 10% Glycerin, 10% Isopropanol + 20% Essigsäureethylester

Es wurde festgestellt, dass die rekombinate Alkohol-Dehydrogenase aus L minor in der im Zwei-Phasen-System verwendeten Kombination von Lösungsmitteln mehrere Tage stabil und aktiv ist.

## Patentansprüche

1. Verfahren zur enantioselektiven Reduktion einer Ketoverbindung der Formel I
R¹-C(O)-R² (I)
wobei R¹ und R² unabhängig voneinander gleich oder verschieden sind und für
1. Wasserstoffatom,
2. -(C₁-C₂₀)-Alkyl, worin Alkyl geradkettig oder verzweigt ist,
3. -(C₂-C₂₀)-Alkenyl, worin Alkenyl geradkettig oder verzweigt ist und gegebenenfalls ein, zwei, drei oder vier Doppelbindungen enthält,
4. -(C₂-C₂₀)-Alkinyl, worin Alkinyl geradkettig oder verzweigt ist und gegebenenfalls ein, zwei, drei oder vier Dreifachbindungen enthält,
5. -(C₆-C₁₄)-Aryl,
6. -(C₁-C₈)=Alkyl-(C₆-C₁₄)-Aryl, oder
7. R¹ und R² bilden zusammen mit dem -C(O)-Rest ein -(C₆-C₁₄)-Aryl oder einen -(C₅-C₁₄)-Heterocyclus,
wobei die oben unter 1. bis 7. genannten Reste unsubstituiert sind oder ein- bis dreifach substituiert sind unabhängig voneinander durch
a) -OH,
b) Halogen, wie Fluor, Chlor, Brom oder Jod,
c) -NO₂,
d) -C(O)-O-(C₁-C₂₀)-Alkyl, worin Alkyl gerade oder verzweigt ist und unsubstituiert oder ein- bis dreifach substituiert ist durch Halogen, Hydroxyl, Amino oder Nitro, oder
e) -(C₅-C₁₄)-Heterocyclus, der unsubstituiert oder ein- bis dreifach substituiert ist durch Halogen, Hydroxyl, Amino oder Nitro,
steht, **dadurch gekennzeichnet, dass**
a) eine Verbindung der Formell mit einem Anteil von 10% bis 25%, bezogen auf das Gesamtvolumen des Reaktionsansatzes, Alkoholdehydrogenase, Wasser, Cofaktor NADPH oder NADH und ein organisches Lösungsmittel mit einem IogP von 0,5 bis 4,0;
b) in einem Zwei-Phasensystem aus Wasser, organischem Lösungsmittel und der Verbindung der Formel (I) inkubiert wird;
c) der durch die Alkoholdehydrogenase gebildete oxidierte Cofaktor kontinuierlich regeneriert, und
d) die chirale Hydroxyverbindung isoliert wird.

2. Verfahren gemäß Anspruch 1, **dadurch gekennzeichnet, dass** eine Verbindung der Formel I aus der Reihe 4-Chlor-3-oxo- butansäureethylester, Acetophenon, Acetessigsäuremethylester, Ethyl-2-oxo-4-phenylbutyrat, 2,5- Hexandion, Ethylpyruvat oder 2-Octanon eingesetzt wird.

3. Verfahren gemäß Anspruch 1 oder 2, **dadurch gekennzeichnet, dass** ein organisches Lösungsmittel mit einem IogP von 0,6 bis 3,0, insbesondere von 0,6 bis 1,9, eingesetzt wird.

4. Verfahren gemäß Anspruch 3, **dadurch gekennzeichnet, dass** ein organisches Lösungsmittel mit einem IogP von 0,63 bis 1,75 eingesetzt wird.

5. Verfahren gemäß Anspruch 1, **dadurch gekennzeichnet, dass** als organisches Lösungsmittel Diethylether, tertiär-Butylmethylether, Diisopropytether oder Essigsäureethylester eingesetzt wird.

6. Verfahren gemäß einem oder mehreren der Ansprüche 1 bis 5, **dadurch gekennzeichnet, dass** eine Alkohol-Dehydrogenasen aus Hefe, Pferdeleber, Thermoanaerobium brockii, Rhodococcus erythropolis, Lactobacillus kefir, Lactobacillus brevis, Lactobacillus minor oder eine Alkohol-Dehydrogenase mit der Aminosäuresequenz gemäß SEQ ID NO: 4 eingesetzt wird.

7. Verfahren gemäß einem oder mehreren der Ansprüche 1 bis 6, **dadurch gekennzeichnet, dass** ein Puffer wie Kaliumphosphat-, Tris/HCl- oder Triethanolamin-Puffer mit einem pH-Wert von 5 bis 10, bevorzugt mit einem pH-Wert von 6 bis 9, zugesetzt wird.

8. Verfahren gemäß Anspruch 7, **dadurch gekennzeichnet, dass** dem Puffer Magnesiumionen wie MgCl₂, in einer Konzentration von 0,2 mM bis 10 mM, bevorzugt 0,5 mM bis 2 mM, zugesetzt werden.

9. Verfahren gemäß einem oder mehreren der Ansprüche 1 bis 8, **dadurch gekennzeichnet, dass** als Cofaktor NADPH oder NADH in einer Menge von 0,05 mM bis 0,25 mM, insbesondere von 0,06 mM bis 0,2 mM, bezogen auf die wässrige Phase, zugesetzt wird.

10. Verfahren gemäß einem oder mehreren der Ansprüche 1 bis 9, **dadurch gekennzeichnet, dass** als Stabilisator für die Alkohol-Dehydrogenase Glycerin, Sorbitol oder Dimethylsulfoxid zugefügt wird.

11. Verfahren gemäß einem oder mehreren der Ansprüche 1 bis 10, **dadurch gekennzeichnet, dass** Isopropanol zugefügt wird.

12. Verfahren gemäß einem oder mehreren der Ansprüche 1 bis 11, **dadurch gekennzeichnet, dass** die Verbindungen der Formel I in einer Menge von 15% bis 22 %, bezogen auf das Gesamtvolumen, , eingesetzt werden.

13. Verfahren gemäß einem oder mehreren der Ansprüche 1 bis 12, **dadurch gekennzeichnet, dass** die Umsetzung bei einer Temperatur von etwa 10 °C bis 70 °C, bevorzugt von 30 °C bis 60 °C, durchgeführt wird.

14. Verfahren gemäß einem oder mehreren der Ansprüche 1 bis 13, **dadurch gekennzeichnet, dass** das organische Lösungsmittel in einer Menge von1 % bis 90 %, bezogen auf das Gesamtvolumen des Reaktionsansatzes, vorzugsweise von 15 % bis 60 %, insbesondere von 20 % bis 50 %, eingesetzt werden.

15. Verfahren gemäß einem oder mehreren der Ansprüche 1 bis 14, **dadurch gekennzeichnet, dass** das Verhältnis von organischen Lösungsmittel zu Wasser von 9 zu 1 bis 1 zu 9, vorzugsweise von 1 zu 1 bis 1 zu 3, beträgt.

16. Verfahren gemäß Anspruch 10, **dadurch gekennzeichnet, dass** der Stabilisator in einer Menge von 5 % bis 30 %, bezogen auf das Volumen des gesamten Reaktionsansatzes, bevorzugt von 10 % bis 20 %, insbesondere 20 %, eingesetzt wird.

17. Verfahren gemäß Anspruch 11, **dadurch gekennzeichnet, dass** Isopropanol in einer Menge von 5 % bis 30 %, bezogen auf das Volumen des gesamten Reaktionsansatzes, bevorzugt von 10 % bis 20 %, insbesondere 10 %, eingesetzt wird.

18. Verfahren gemäß Anspruch 6, **dadurch gekennzeichnet, dass** die Alkohol-Dehydrogenase in einer Menge von 20 000 U bis 200 000 U pro kg umzusetzender Verbindung der Formel I, bevorzugt etwa 100 000 U, eingesetzt wird.

## Claims

1. A method for the enantioselective reduction of a keto compound of formula I
R¹-C(O)-R² (I)
wherein R¹ and R² are, independently of each other, identical or different and are
1. a hydrogen atom,
2. -(C₁-C₂₀)-alkyl in which alkyl is straight-chained or branched,
3. -(C₂-C₂₀)-alkenyl in which alkenyl is straight-chained or branched and, optionally, comprises one, two, three or four double bonds,
4. -(C₂-C₂₀)-alkynyl in which alkynyl is straight-chained or branched and, optionally, comprises one, two, three or four triple bonds,
5. -(C₆-C₁₄)-aryl,
6. -(C₁-C₈)-alkyl-(C₆-C₁₄)-aryl, or
7. R¹ and R² form in combination with the -C(O) radical a -(C₆-C₁₄)-aryl or a -(C₅-C₁₄)-heterocycle,
wherein the radicals defined above under 1. to 7. are unsubstituted or, independently of each other, mono- to trisubstituted by
a) -OH,
b) halogen such as fluorine, chlorine, bromine or iodine,
c) -NO₂
d) -C(O)-O-(C₁-C₂₀)-alkyl in which alkyl is linear or branched and unsubstituted or mono- to trisubstituted by halogen, hydroxyl, amino or nitro, or
e) -(C₅-C₁₄)-heterocycle which is unsubstituted or mono- to trisubstituted by halogen, hydroxyl, amino or nitro,
**characterized in that**
a) a compound of formula I with a proportion of from 10% to 25%, based on the total volume of the reaction batch, alcohol dehydrogenase, water, cofactor NADPH or NADH and an organic solvent having a IogP of from 0.5 to 4.0;
b) are incubated in a two-phase system of water, organic solvent and the compound of formula (I);
c) the oxidized cofactor produced by said alcohol dehydrogenase is continuously regenerated, and
d) the chiral hydroxy compound is isolated.

2. A method according to claim 1, **characterized in that** a compound of formula I of the group 4-chloro-3-oxo-butanoic acid ethyl ester, acetophenone, methyl acetoacetate, ethyl-2-oxo-4-phenylbutyrate, 2,5-hexanedione, ethyl pyruvate or 2-octanone is used.

3. A method according to claim 1 or 2, **characterized in that** an organic solvent having a logP of from 0.6 to 3.0, in particular from 0.6 to 1.9, is used.

4. A method according to claim 3, **characterized in that** an organic solvent having a logP of from 0.63 to 1.75 is used.

5. A method according to claim 1, **characterized in that** the organic solvent used is diethyl ether, tert-butyl methyl ether, diisopropyl ether or ethyl acetate.

6. A method according to one or several of claims 1 to 5, **characterized in that** an alcohol dehydrogenase from yeast, horse liver, Thermoanaerobium brockii, Rhodococcus erythropolis, Lactobacillus kefir, Lactobacillus brevis, Lactobacillus minor or an alcohol dehydrogenase having the amino acid sequence according to SEQ ID NO: 4 is used.

7. A method according to one or several of claims 1 to 6 **characterized in that** a buffer such as potassium phosphate, Tris/HCl or triethanolamine buffer having a pH of from 5 to 10, preferably having a pH of from 6 to 9, is added.

8. A method according to claim 7, **characterized in that** magnesium ions such as MgCl₂ at a concentration of from 0.2 mM to 10 mM, preferably from 0.5 mM to 2 mM, are added to the buffer.

9. A method according to one or several of claims 1 to 8, **characterized in that** the cofactor added is NADPH or NADH in an amount of from 0.05 mM to 0.25 mM, in particular from 0.06 mM to 0.2 mM, based on the aqueous phase.

10. A method according to one or several of claims 1 to 9, **characterized in that** glycerol, sorbitol or dimethyl sulfoxide is added as a stabilizer for alcohol dehydrogenase.

11. A method according to one or several of claims 1 to 10, **characterized in that** isopropanol is added.

12. A method according to one or several of claims 1 to 11, **characterized in that** the compounds of formula I are used in an amount of from 15% to 22%, based on the total volume.

13. A method according to one or several of claims 1 to 12, **characterized in that** the reaction is carried out at a temperature of from about 10°C to 70°C, preferably from 30°C to 60°C.

14. A method according to one or several of claims 1 to 13, **characterized in that** the organic solvent is used in an amount of from 1 % to 90%, based on the total volume of the reaction batch, preferably from 15% to 60%, in particular from 20% to 50%.

15. A method according to one or several of claims 1 to 14, **characterized in that** the ratio of organic solvent to water is from 9 to 1 to 1 to 9, preferably from 1 to 1 to 1 to 3.

16. A method according to claim 10, **characterized in that** the stabilizer is used in an amount of from 5% to 30%, based on the volume of the total reaction batch, preferably from 10% to 20%, in particular 20%.

17. A method according to claim 11, **characterized in that** isopropanol is used in an amount of from 5% to 30%, based on the volume of the total reaction batch, preferably from 10% to 20%, in particular 10%.

18. A method according to claim 6, **characterized in that** the alcohol dehydrogenase is used in an amount of from 20 000 U to 200 000 U per kg of compound of formula I to be reacted, preferably of about 100 000 U.

## Revendications

1. Procédé pour la réduction énantiosélective d'un composé céto de formule I
R¹-C(O)-R² (I)
dans laquelle R¹ et R² indépendamment l'un de l'autre sont identiques ou différents et représentent
1. un atome d'hydrogène,
2. un group alkyle en C₁ à C₂₀, dans lequel le group alkyle est à chaîne droite ou ramifié,
3. un groupe alcényle en C₂ à C₂₀, dans lequel le groupe alcényle est à chaîne droite ou ramifié, et contient éventuellement une, deux, trois ou quatre double(s) liaison(s),
4. un groupe alcynyle en C₂ à C₂₀, dans lequel le groupe alcynyle est à chaîne droite ou ramifié, et contient éventuellement une, deux, trois ou quatre triple(s) liaison(s),
5. un groupe aryle en C₆ à C₁₄,
6. un groupe (alkyle en C₁ à C₈) - (aryle en C₈ à C₁₄) , ou
7. R¹ et R² forment ensemble avec le radical -C(O)-un groupe aryle en C₆ à C₁₄ ou un hétérocycle en C₅ à C₁₄,
les radicaux cités ci-dessus en 1. à 7. étant non substitués ou étant substitués une à trois fois, indépendamment l'un de l'autre par
a) un groupe OH,
b) un atome d'halogène, comme un atome de fluor, de chlore, de brome ou d'iode,
c) un groupe NO₂,
d) un groupe -C(O)-O-(alkyle en C₁ à C₂₀, dans lequel le groupe alkyle est linéaire ou ramifié et non substitué, ou une à trois fois substitué par un atome d'halogène, un groupe hydroxyle, amino ou nitro, ou
e) un hétérocycle en C₅ à C₁₄, qui est non substitué ou substitué une à trois fois par un atome d'halogène, un groupe hydroxyle, amino ou nitro,
**caractérisé en ce que**,
a) un composé de formule I ayant une proportion de 10% à 25%, par rapport au volume total du mélange réactionnel, d'alcool déshydrogénase, d'eau, de cofacteur NADPH ou NADH et un solvant organique ayant un logP de 0,5 à 4,0 ;
b) est incubé dans un système biphasique constitué d'eau, d'un solvant organique et du composé de formule (I) ;
c) qui régénère de façon continue les cofacteurs oxydés formés par l'alcool déshydrogénase, et
d) le composé hydroxy chiral est isolé.

2. Procédé selon la revendication 1, **caractérisé en ce qu'**un composé de formule I de la série comprenant l'ester éthylique de l'acide 4-chloro-3-oxo-butanoïque, l'acétophénone, l'ester méthylique de l'acide acétique, le butyrate d'éthyl-2-oxo-4-phényle, la 2,5-hexanedione, le pyruvate d'éthyle ou la 2-octanone, est mis en oeuvre.

3. Procédé selon la revendication 1 ou 2, **caractérisé en ce qu'**un solvant organique ayant un logP de 0,6 à 3,0, en particulier de 0,6 à 1,9 est mis en oeuvre.

4. Procédé selon la revendication 3, **caractérisé en ce qu'**un solvant organique ayant un logP de 0,63 à 1,75 est mis en oeuvre.

5. Procédé selon la revendication 1, **caractérisé en ce que** l'éther diéthylique, l'éther tert-butylméthylique, l'éther diisopropylique ou l'ester d'éthyle de l'acide acétique sont mis en oeuvre en tant que solvant organique.

6. Procédé selon l'une ou plusieurs des revendications 1 à 5, **caractérisé en ce qu'**une alcool déshydrogénase issue de levure, de foie de cheval, de *Thermoanerobium brockii, Rhodococcus erythropolis, Lactobacillus kefir, Lactobacillus brevis, Lactobacillus minor* ou une alcool déshydrogénase ayant la séquence d'acides aminés selon SEQ ID NO :4, est mise en oeuvre.

7. Procédé selon l'une ou plusieurs des revendications 1 à 6, **caractérisé en ce qu'**un tampon comme un tampon au phosphate de potassium, au Tris/HCl ou à la triéthanolamine avec une valeur de pH de 5 à 10, de préférence avec une valeur de pH de 6 à 9, est ajouté.

8. Procédé selon la revendication 7, **caractérisé en ce que** des ions magnésium comme MgCl₂, sont ajoutés au tampon en une concentration de 0,2 mM à 10 mM, de préférence 0,5 mM à 2 mM.

9. Procédé selon l'une ou plusieurs des revendications 1 à 8, **caractérisé en ce que** NADPH ou NADH est ajouté en tant que cofacteur en une quantité de 0,05 mM à 0,25 mM, en particulier de 0,06 mM à 0,2 mM, par rapport à la phase aqueuse.

10. Procédé selon l'une ou plusieurs des revendications 1 à 9, **caractérisé en ce que** le glycérol, le sorbitol ou le diméthylsulfoxyde sont ajoutés en tant que stabilisant pour l'alcool déshydrogénase.

11. Procédé selon l'une ou plusieurs des revendications 1 à 10, **caractérisé en ce que** l'isopropanol est ajouté.

12. Procédé selon l'une ou plusieurs des revendications 1 à 11, **caractérisé en ce que** les composés de formule I sont mis en oeuvre en une quantité de 15% à 22%, par rapport au volume total.

13. Procédé selon l'une ou plusieurs des revendications 1 à 12, **caractérisé en ce que** la réaction est conduite à une température d'environ 10°C à 70°C, de préférence de 30°C à 60°C.

14. Procédé selon l'une ou plusieurs des revendications 1 à 13, **caractérisé en ce que** le solvant organique est mis en oeuvre en une quantité de 1% à 90%, par rapport au volume total du mélange réactionnel, de préférence de 15 à 60%, en particulier de 20% à 50%.

15. Procédé selon l'une ou plusieurs des revendications 1 à 14, **caractérisé en ce que** le rapport de solvant organique à l'eau est de 9 à 1 jusqu'à 1 à 9, de préférence de 1 à 1 jusqu'à 1 à 3.

16. Procédé selon la revendication 10, **caractérisé en ce que** le stabilisant est mis en oeuvre en une quantité de 5% à 30%, par rapport au volume du mélange réactionnel total, de préférence de 10% à 20%, en particulier 20%.

17. Procédé selon la revendication 11, **caractérisé en ce que** le propanol est mis en oeuvre en une quantité de 5% à 30%, par rapport au volume du mélange réactionnel total, de préférence de 10% à 20%, en particulier 10%.

18. Procédé selon la revendication 6, **caractérisé en ce que** l'alcool déshydrogénase est mise en oeuvre en une quantité de 20 000 U à 200 000 U par kg de composé de formule I à faire réagir, de préférence environ 100 000 U.


### SEQUENZ PROTOKOLL

<110> Juelich Enzyme Products GmbH
<120> Enzymatisches Verfahren zur enantioselektiven Reduktion von Ketoverbindungen
<130> (TH) Juelich Enzyme
<140>
   <141>
<160> 4
<170> PatentIn Ver. 2.1
<210> 1
   <211> 795
   <212> DNA
   <213> Künstliche Sequenz
<220>
   <223> Eeschreibung der künstlichen Sequenz:DNA Primer
<400> 1
   atgagaggat cgcatcacca tcaccatcac ggatccatga ccgatcggtt gaaggggaaa 60
   gtagcaattg taactggcgg tacctcggca attggcttgg caatcgctga taagtttgtt 120
   gaagaaggcg caaaggttgt tattaccggc cgtcacgctg atgtaggtga aaaagctgcc 180
   agatcaatcg gcggcacaga cgttatccgt tttgtccaac acgatgcttc tgatgaaacc 240
   ggctggacta agttgtttga tacgactgaa gaagcatttg gcccagttac cacggttgtc 300
   aacaatgccg gaattgcggt cagcaagagt gttgaagata ccacaactga agaatggcgc 360
   aagctgctct cagttaactt ggatggtgtc ttcttcggta cccgtcttgg aatccaacgt 420
   atgaagaata aaggactcgg agcatcaatc atcaatatgt catctatcga aggttttgtt 480
   ggtgatccag ctctgggtgc atacaacgct tcaaaaggtg ctgtcagaat tatgtctaaa 540
   tcagctgcct tggattgcgc tttgaaggac tacgatgttc gggttaacac tgttcatcca 600
   ggttatatca agacaccatt ggttgacgat cttgaagggg cagaagaaat gatgtcacag 660
   cggaccaaga caccaatggg tcatatcggt gaacctaacg atatcgcttg gatctgtgtt 720
   tacctggcat ctgacgaatc taaatttgcc actggtgcag aattcgttgt cgacggaggg 780
   tacaccgccc aatag                                                  795
<210> 2
   <211> 37
   <212> DNA
   <213> Künstlich Sequenz
<220>
   <223> Beschreibung der künstlichen Sequenz:DNA Primer
<400> 2
   gcggatccat gacngaycgn ttraarggna argtngc 37
<210> 3
   <211> 36
   <212> DNA
   <213> Künstlich Sequenz
<220>
   <223> Beschreibung der künstlichen Sequenz:DNA Primer
<400> 3
   gggaagcttc Laytgngcng trtanccncc rtcnac 36
<210> 4
   <211> 264
   <212> PRT
   <213> Lactcbacillus sp.
<400> 4
   Met Arg Gly Ser His His His His His His Gly Ser Met Thr Asp Arg
   1               5                   10                  15
   Leu Lys Gly Lys Val Ala Ile Val Thr Gly Gly Thr Leu Gly Ile Gly
               20                  25                  30
   Leu Ala Ile Ala Asp Lys Phe Val Glu Glu Gly Ala Lys Val Val Ile
           35                  40                  45
   Thr Gly Arg His Ala Asp Val Gly Glu Lys Ala Ala Arg Ser Ile Gly
       50                  55                  60
   Gly Thr Asp Val Ile Arg Phe Val Gln His Asp Ala Ser Asp Glu Thr
   65                  70                  75                  80
   Gly Trp Thr Lys Leu Phe Asp Thr Thr Glu Glu Ala Phe Gly Pro Val
                   85                  90                  95
   Thr Thr Val Val Asn Asn Ala Gly Ile Ala Val Ser Lys Ser Val Glu
               100                 105                 110
   Asp Thr Thr Thr Glu Glu Trp Arg Lys Leu Leu Ser Val Asn Leu Asp
           115                 120                 125
   Gly Val Phe Phe Gly Thr Arg Leu Gly Ile Gln Arg Met Lys Asn Lys
           130                     135                     140
   Gly Leu Gly Ala Ser Ile Ile Asn Met Ser Ser Ile Glu Gly Glu Val
   145                 150                 155                 160
   Gly Asp Pro Ala Leu Gly Ala Tyr Asn Ala Ser Lys Gly Ala Val Arg
                   165                 170                 175
   Ile Met Ser Lys Ser Ala Ala Leu Asp Cys Ala Leu Lys Asp Tyr Asp
               180                 185                 190
   Val Arg Val Asn Thr Val His Pro Gly Tyr Ile Lys Thr Pro Leu Val
           195                 200                 205
   Asp Asp Leu Glu Gly Ala Glu Glu Met Met Ser Gln Arg Thr Lys Thr
       210                 215                 220
   Pro Met Gly His Ile Gly Glu Pro Asn Asp Ile Ala Trp Ile Cys Val
   225                 230                 235                 240
   Tyr Leu Ala Ser Asp Glu Ser Lys Phe Ala Thr Gly Ala Glu Phe Val
                   245                 250                 255
   Val Asp Gly Gly Tyr Thr Ala Gln
               260